# EUROPÄISCHE PATENTSCHRIFT

(11) **EP 2 418 269 B1**
(45) Veröffentlichungstag und Bekanntmachung des Hinweises auf die Patenterteilung: **03.07.2013**
(21) Anmeldenummer: 11177339.6
(22) Anmeldetag: 11.08.2011
(51) Int. Cl.: C12M 3/04

(54) **Perfusionsvorrichtung**
Perfusion device
Dispositif de perfusion

(30) Priorität: 11.08.2010 DE 102010039229
(43) Veröffentlichungstag der Anmeldung: 15.02.2012
(73) Patentinhaber: Universität Potsdam, 14469 Potsdam (DE)
(72) Erfinder: Gerhardt, Matthias, 16348 Wandlitz (DE); Beta, Carsten, 12163 Berlin (DE)
(74) Vertreter: Müller & Schubert

(56) Entgegenhaltungen:
- WO-A2-2008/108746
- WO-A2-2009/141163
- DE-A1- 10 032 808
- US-A- 4 241 187
- US-A1- 2006 032 746
- US-A1- 2007 224 677

## Beschreibung

Die Erfindung betrifft eine Vorrichtung zur multilokalen und raumzeitlich definierten Perfusion des Innenvolumens eines Gefäßes.

Im Stand der Technik sind eine Reihe von gattungsgemäßen Perfusionsvorrichtungen beschrieben.

In Zibek et al. (Zibek S., Stett A, Koltay P Hu M., Zengerle R., Nisch W, Stelzle M. "Localized functional synthesis and characterization of TE 671 cells cultured on nanoporous membrane by calcein and acetylcholine", Biophys J., 92(1):L04-6., (2007)), wird eine Apparatur beschrieben, bei welcher mittels Ink-Jet (Tintenstrahldrucker) eine aktive Substanz auf die unten liegende Seite einer mikro- oder nano- porösen Membran gedruckt wird. Auf der oben liegenden Seite der Membran befindet sich ein Kulturgefäß, in welchem eine Nährlösung zusammen mit einzelnen Zellen oder ein Gewebeverbund mit der Membran in Kontakt stehen. Die mittels Ink-Jet aufgedruckte aktive Substanz diffundiert durch die Membran hindurch und tritt sodann mit den Zellen oder dem Gewebeverbund in Interaktion.

Wang et al. (Wang HY., Bao N., Lu C. "A microfluidic cell array with individually addresable culture chambers", Biosensors and Bioelectronics 24 (2008) 613-617) beschreiben ein Array aus Kammern, welche über Kanäle adressierbar sind. Diese Kanäle können an externe Versorgungseinrichtungen angeschlossen werden. In den Kammern können Mikroorganismen oder Zellen wachsen und über die Kanäle mit Nährstoffen oder mit anderen Substanzen perfundiert werden.

Peterman et al. (Peterman MC., Nooland J., Blumenkranz MS., Fishman H. "Localized chemical release from artificial synapse chip", PNAS 6, (2004) 9951-9954)beschreiben ein Array, aus Löchern in einer Siliziumnitrid (SiNi) Membran, die von einer Seite her durch separate Kanäle ständig mit Perfusionssubstanz umströmt sind und auf der anderen Seite mit einem Kulturgefäß in Kontakt stehen. In dem Kulturgefäß befinden sich entweder Zellen oder ein Gewebeverbund. Ein elektrisches Feld welches sich von der einen Seite zur anderen Seite der SiNi Membran erstreckt, bewirkt den Transport der Perfusionssubstanz durch die Löcher hindurch mittels Elektroosmose. Je nach Polarität des Feldes kann so ein Substanzstrom eingeschaltet oder ausgeschaltet werden.

In der DE 10032808 A1 wird eine Vorrichtung zur Zusammenführung unterschiedlicher Zelllinien in bestimmten Formen und Mustern zu Geweben und Organen beschrieben, wie sie beispielsweise bei der Haut auftreten.

Die US-A 20030032946 beschreibt künstliche Synapsen-Chips, welche eine Verbindung zwischen dem Zellprozess und einer chemischen oder elektrischen Vorrichtung zur neuronalen Anregung herstellt.

Die US-A 20070224677 offenbart Mikrogefäß-Systeme, die in Vorrichtungen integriert sind, die eine kontrollierte Perfusion mit Fluiden gestatten.

In der US-A 20090317787 sind Mikrogefäß-Systeme beschrieben, welche perfundierbar sind und Dorne aufweisen, welche die fluidische Verbindung zwischen verschiedenen Bereichen bewirken.

Die US-A 20060032746 betrifft Verfahren und Vorrichtungen zum Inkontaktbringen von mikrofluidischen Strukturen, wobei beispielsweise eine Hohlnadel verwendet wird.

Die aus dem Stand der Technik bekannten Perfusionsvorrichtungen weisen Nachteile auf. Vorrichtungen nach dem Stand der Technik basieren auf Elektroosmose, welche auf Basis eines elektrischen Feldes funktioniert. Dieses elektrische Feld könnte bei Substanzen, Zellen oder Geweben zu unerwünschten Wechselwirkungen führen.

Zur Vermeidung dieses Problems machen sich andere Vorrichtungen das Prinzip des Ink-Jet(Tintenstrahldruckers) zunutze. Damit der Ink-Jet Perfusionssubstanzen freisetzen kann, werden Teile der Perfusionssubstanz einerseits ultrahoch erhitzt und anderseits muss ein Luftspalt an der Öffnung des Ink-Jet vorgesehen sein, damit dieser überhaupt etwas freisetzten kann. Beides könnte bestimmte Substanzen durch Hitzeschädigung oder Luftkontakt verändern.

Ein weiterer Nachteil, welcher bei Vorrichtungen nach dem Stand der Technik besteht, hat mit dem Anschließen sehr kleiner Kammern und Kanäle an externe Versorgungskomponenten zu tun. Solche Vorrichtungen sind bisher dadurch gekennzeichnet, dass Hohlnadeln zum Einstich in das Substrat verwendet werden. Damit die Öffnung der Hohlnadel mit den Mikrokanälen hydraulisch überhaupt verbunden werden kann, müssen Kavernen mit erheblich größerem Querschnitt als die Mikrokanäle vorgesehen sein. Diese können leicht durch die Hohlnadel getroffen werden. Leider kann in der Kaverne ein Luftbläschen entstehen, welches in den Mikrokanälen und überhaupt zu erheblichen Problemen führen könnte (Verstopfung, Druckschwankung).

In der WO 2010/101708 A2 wird ein System zur Untersuchung der Einwirkung verschiedenster Substanzen auf zelluläre oder allgemein biologische Untersuchungsobjekte beschrieben. Das Gerät besteht aus einem Mikrokanal, der unter anderem eine Perforation in Richtung eines Zellkulturgefäßes aufweist, die zugleich auch als Quelle oder "Perfusionsport" zum Heranführen der Testsubstanzen mit konstanter oder zeitlich variabler Konzentration an die zellulären oder biologischen Untersuchungsobjekte dient. Bevorzugt ist dabei eine Anordnung, bei der die Untersuchungsobjekte in eine Matrix eingebettet sind, die entweder aus einem flüssigem oder ebenfalls einem bevorzugt gelförmigen Material besteht. In einer besonderen Ausführungsform ist das Kulturgefäß zu dessen offener Seite hin mit einem "cover slip" abgedeckt, wobei sich die zellulären Untersuchungsobjekte zwischen der Oberfläche der Matrix und dem "cover slip" befinden können.

In der US 2009/0023608 A1 ist ein Zellkultursystem offenbart, dessen Zweck die Perfusion von zellulären oder allgemein biologischen Untersuchungsobjekten mit Testsubstanzen ist. Eine als Kulturgefäß definierte Kammer ist durch einen Mikrokanal teilweise umgeben, wobei das Kammervolumen mit dem Kanal durch Perforationen hindurch in Verbindung steht. Dadurch können Substanzen zwischen dem Kanal und dem Kammervolumen durch Konvektion und Diffusion ausgetauscht werden. Bevorzugt ist hierbei ein im Speziellen so bemessener Querschnitt der Perforationen, dass Diffusion über Konvektion bezüglich des Substanztransportmechanismus zwischen Kammervolumen und Kanal überwiegt.

In DE 10 2007 034 935 A1 wird ein (Bio)Sensorsystem für verschiedene Umgebungszustandsgrößen benannt, dessen Messgröße entweder ein Elektrodenstrom oder ein Elektrodenpotential ist. Die Elektroden sind daher bevorzugt in einer Messkammer untergebracht und ferner bevorzugt durch einen Elektrolyt, der auch als "Transfermedium" bezeichnet wird, umgeben. Zu einer Seite hin ist die Messkammer mit einer Membran abgeschlossen, deren maßgebliche Eigenschaft als Konvektionsbarriere zu wirken, sich mit [0039] erschließt: "jedoch die Konvektion der Medien durch die Membran hindurch weites gehend behindert". Die Membran trennt somit Untersuchungsobjekte und Sensor, erlaubt aber dennoch den Übertritt von Substanzen, die in enger Verbindung mit der Messgröße stehen.

Die Aufgabe der vorliegenden Erfindung ist es, die oben genannten Nachteile zu umgehen, um z. B. Gewebeverbände oder einzelne Zellen multilokal raumzeitlich mit einer Vielzahl an Substanzen zu perfundieren. Hierdurch können zum Beispiel Effekte von Substanzen auf Zellen oder Gewebe untersucht werden.

Die Aufgabe wird durch eine Perfusionsvorrichtung mit den Merkmalen des Hauptanspruchs gelöst. Vorteilhafte Weiterbildungen der Erfindung sind in den abhängigen Unteransprüche gekennzeichnet. Im folgenden wird die erfindungsgemäße Perfusionsvorrichtung als Mikroperfusionsarray bezeichnet und mit MPA abgekürzt.

Gegenstand der vorliegenden Erfindung ist somit eine Perfusionsvorrichtung zum Perfundieren von biologischem Material, umfassend
- ein Trägersubstrat mit mindestens einem darin befindlichen Kanal,
- eine auf dem Trägersubstrat flächig angeordnete Diffusionsbarriere, welche die Oberseite der im Trägersubstrat angeordneten Kanäle abdeckt, wobei die Diffusionsbarriere im Bereich der Kanäle mit mindestens einer Perforation versehen ist
- eine auf der Diffusionsbarriere flächig angeordnete Konvektionsbarriere,
- je Kanal mindestens je zwei Anschlüsse zum Verteilen von Fluiden in dem Kanal.

Dabei ist es bevorzugt, dass auf der Oberseite der Konvektionsbarriere eine weitere Substratschicht flächig angeordnet ist, wobei in der Substratschicht im Bereich der Perforation der Diffusionsbarriere mindestens eine Öffnung vorgesehen ist.

Weiterhin ist bevorzugt, dass auf einer Seite der Konvektionsbarriere im wesentlichen konzentrisch zu den Perforationen in der Diffusionsbarriere ein Hohlzylinder als "Kulturgefäß" angeordnet ist.

Bevorzugt ist es ferner, dass das Trägersubstrat und/oder die weitere Substratschicht aus einem flexiblen Material bestehen, welches gegen die Fluide im Kanal chemisch beständig ist und dass die Diffusionsbarriere aus einem flexiblen oder starren Material besteht, welches gegen die Fluide im Kanal chemisch beständig ist.

Bevorzugt ist ferner, dass die Konvektionsbarriere aus einem Material besteht, welches gegen die Fluide im Kanal oder im Kulturgefäß chemisch beständig ist. Dabei ist besonders bevorzugt, dass die Konvektionsbarriere aus einem mikro- oder nanoporösem Material besteht. Die Poren verbinden zwei gegenüberliegende Seiten der Konvektionsbarriere und sind durch einen definierten Durchmesser gekennzeichnet. Die Poren können außerdem mit einer materiellen (Innen-)Beschichtung ausgestattet sein, die mit zur Anwendung kommenden Flüssigkeiten oder darin gelösten Substanzen elektrostatisch oder mechanisch (durch Reibung) wechselwirkt.

In einer weiteren bevorzugten Ausführungsform der vorliegenden Erfindung ist eine erfindungsgemäße Perfusionsvorrichtung dadurch gekennzeichnet, dass in den Fluiden im Kanal und/oder im Kulturgefäß dispergierte Partikel vorgesehen sind, welche an der Konvektionsbarriere eine statische oder transiente Druckdifferenz ausbilden und wobei in der Konvektionsbarriere Poren ausgebildet sind, deren Querschnitt derart gewählt ist, dass der Durchtritt der dispergierten Partikel verhindert, der Durchtritt von Perfusionssubstanzen jedoch ermöglicht ist.

Die Konvektionsbarriere kann somit als semipermeable Membran angesehen werden. Entscheidend ist, dass eine Durchlässigkeit der Barriere von der Größe (dem Durchmesser) der Partikel abhängig ist.

Dabei ist es besonders bevorzugt, dass die in den Fluiden vorgesehenen dispergierten Partikel aus ferromagnetischen und/oder kolloidalen Partikeln ausgewählt sind.

Weiterhin ist erfindungsgemäß hierbei besonders bevorzugt, dass die Perfusionsvorrichtung in einem schaltbaren magnetischen Feld angeordnet ist. Das magnetische Feld ist erfindungsgemäß in der Feldstärke und in der Ausrichtung des Feldes regelbar.

Ein weiterer Gegenstand der vorliegenden Erfindung ist ein Verfahren zum Perfundieren von biologischem Material, wobei man
in einer Perfusionsvorrichtung, welche eine mit Poren versehene Konvektionsbarriere aufweist, welche auf der einen Oberfläche das biologische Material trägt und auf der gegenüberliegenden Oberfläche mit einem Transportfluid in fluidischer Kommunikation ist,
das Transportfluid mit dispergierten Partikeln versieht, welche an der Konvektionsbarriere eine Druckdifferenz ausbilden, da die Poren der Konvektionsbarriere einen Durchmesser aufweisen, der kleiner ist, als der Durchmesser der dispergierten Partikel,
dem Transportfluid eine raumzeitlich definierte Menge einer Perfusionssubstanz zusetzt und diese in Richtung auf den Bereich der fluidischen Kommunikation leitet,
und wobei die Perfusionssubstanz beim Erreichen des Bereichs der fluidischen Kommunikation mittels der herrschenden Druckdifferenz durch die Konvektionsbarriere hindurch zum biologischen Material strömt.

Dabei ist es bevorzugt, dass man
im Transportfluid als dispergierte Partikel kolloidale Partikel verwendet und
das biologische Material in eine Nährlösung einbringt, welche gleichfalls kolloidale Partikel in einer Menge enthält, die zu einer Druckdifferenz führt, welche den Wert null besitzt.

Dabei ist ferner ganz besonders bevorzugt, dass die im Transportfluid und/oder in der Perfusionssubstanz dispergierten Partikel ferromagnetische Partikel sind, und wobei man wenigstens im Bereich der fluidischen Kommunikation ein schaltbares und in der Stärke und Richtung regulierbares Magnetfeld anlegt, um die ferromagnetischen Partikel zu bewegen. Es ist also erfindungsgemäß bevorzugt, dass die ferromagnetischen Partikel ausschließlich in der Perfusionssubstanz vorliegen. Es ist aber erfindungsgemäß auch bevorzugt, dass die ferromagnetischen Partikel gleichzeitig im Transportfluid und in der Perfusionssubstanz vorliegen.

Ein weiterer Gegenstand der Erfindung ist eine Perfusionsvorrichtung mit einem Werkzeug zum Setzen von Anschlüssen zum Verteilen von Fluiden in einem Kanal, bestehend aus einem im Wesentlichen zylindrischen Körper, der teilweise als Hohlzylinder und teilweise als Vollzylinder ausgebildet ist, wobei der Hohlzylinder als Stanze wirkt und der Vollzylinder eine Bohrung aufweist, welche durch den Boden des zylinderförmigen Körpers in den Vollzylinder führt und aus der Seitenwand des Vollzylinders austritt.

Bevorzugt ist dabei, dass das Werkzeug aus einem Material besteht, welches zum Stanzen der Perfusionsvorrichtung geeignet ist und gegen die Fluide im Kanal chemisch beständig ist. Die Eignung dieses Materials ist dadurch gekennzeichnet, dass beim Stanzen keine plastischen Verformungen am Material auftreten und das Material ferner mit einer Schneidekante (Schnittwinkel >= 10 °) versehen werden kann, die durch Stanzvorgänge nicht beschädigt wird. Dieses Material kann prinzipiell ein metallurgischer Festkörper, bestehend aus allen oder einigen chemischen Elementen, bekannt aus den Nebengruppen des Periodensystems sein. In Spuren können zu Steigerung der Härte, chemischen Beständigkeit und Langlebigkeit zudem auch Elemente der Hauptgruppen 1,2,13,14,15,16 enthalten sein.

Optional befindet sich in dem Werkzeug eine weitere Bohrung, welche vom Boden des Vollzylinders in den Bereich des Hohlzylinders reicht. In dieser Bohrung kann ein beweglicher Stift angeordnet sein, der zum Auswurf von ausgestanztem Material dient.

Gegenstand der Erfindung ist eine Vorrichtung zur raumzeitlich multilokal definierten Perfusion eines Volumens über die temporäre Diffusion von mindestens einer Perfusionssubstanz an mindestens einer Stelle durch eine ebene Konvektionsbarriere hindurch. Die Kontaktzeit der Perfusionssubstanz mit der Konvektionsbarriere wird durch die Fließgeschwindigkeit eines Transportfluides bestimmt. Ein definiertes Volumen der Perfusionssubstanz ist zu diesem Zweck durch das Transportfluid eingeschlossen.

Damit es zur Diffusion der Perfusionssubstanz durch die Konvektionsbarriere kommt, muss ein Konzentrationsgradient über die Konvektionsbarriere bestehen.

Mit dieser Vorrichtung können Zellen oder Gewebe *in vitro* oder *in vivo* multilokal mit einem raumzeitlich definierten Substanzspektrum perfundiert werden. Dies kann entweder der Nährstoffversorgung oder dem Studium der Wechselwirkung zwischen Substanzen z.B. mit Zellen oder Geweben dienen.

Die Probleme des Standes der Technik sind einerseits die Erwärmung oder die Verdunstung der Perfusionssubstanz und andererseits eine mögliche Exposition des perfundierten Objektes durch elektrische Felder oder Joulesche Erwärmung. Erfindungsgemäß werden diese Probleme dadurch vermieden, dass die Perfusionssubstanz durch Diffusion zum perfundierten Objekt aufgrund eines Konzentrationsgradienten gelangt. Die Kontaktzeit zwischen der Perfusionssubstanz und der Konvektionsbarriere definiert die zeitliche Komponente des Konzentrationsgradienten. Die räumliche Komponente des Konzentrationsgradienten wird durch die Anordnung der Öffnungen in der Diffusionsbarriere festgelegt.

Erfindungsgemäß werden diese Probleme weiterhin dadurch vermieden, dass die Perfusionssubstanz durch Konvektion zum perfundierten Objekt aufgrund eines Druckgradienten gelangt. Die Kontaktzeit zwischen der Perfusionssubstanz und der Konvektionsbarriere definiert wie lange Perfusionssubstanz konvektiv zum Objekt transportiert werden kann. Die räumliche Komponente des Druckgradienten wird durch die Anordnung der Öffnungen in der Diffusionsbarriere, durch Konzentrationsunterschiede von dispergierten Partikeln oder durch magnetische Kraftwirkung festgelegt.

Im folgenden wird die Erfindung an Hand der Figuren 1 bis 7 näher beschrieben.

Es zeigt:
Fig. 1 einen Querschnitt durch eine Ausführungsform der erfindungsgemäßen Perfusionsvorrichtung;
Fig. 2 eine Explosionsdarstellung der einzelnen Komponenten einer Ausführungsform der erfindungsgemäßen Perfusionsvorrichtung;
Fig. 3 eine Querschnittsdarstellung der Funktionsweise eines erfindungsgemäßen Stanzwerkzeuges;
Fig. 4 ein Blockschaltbild der prinzipiellen Verwendung der erfindungsgemäßen Perfusionsvorrichtung in zwei unterschiedlichen Arbeitsstellungen;
Fig. 5 einen Querschnitt durch eine weitere Ausführungsform der erfindungsgemäßen Perfusionsvorrichtung mit einer Detailansicht der Konvektionsbarriere im Querschnitt;
Fig. 6 einen Querschnitt durch eine weitere Ausführungsform der erfindungsgemäßen Perfusionsvorrichtung in Unterschiedlichen Arbeitsstellungen; und
Fig. 7 einen Querschnitt durch eine weitere Ausführungsform der erfindungsgemäßen Perfusionsvorrichtung in unterschiedlichen Arbeitsstellungen unter Anwendung magnetischer Felder.

### Aufbau eines MPA:

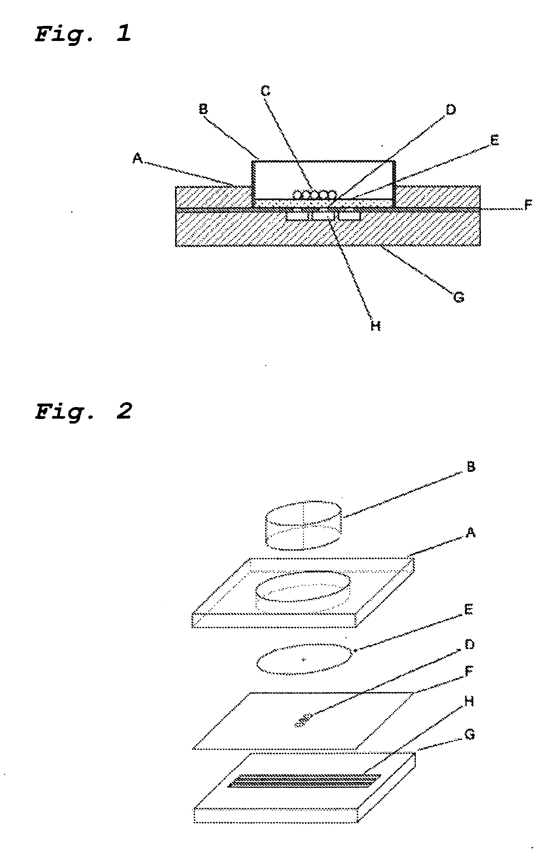

Figur 1 stellt einen Querschnitt durch den Aufbau eines MPA dar, wobei die Kanäle, welche von unterschiedlichen aktiven Substanzen durchflossen werden, in Vorderansicht dargestellt sind.
Figur 2 zeigt die einzelnen Komponenten des MPA in einer Explosionsdarstellung.

Ein Trägersubstrat beinhaltet Kanäle, die zur Oberflachenebene des Substrates hin geöffnet sind. Diese Kanäle werden durch eine ebene Diffusionsbarriere verschlossen. In der Diffusionsbarriere sind Öffnungen vorgesehen, welche mit dem Innenvolumen der Kanäle in Verbindung stehen. Auf die Diffusionsbarriere ist eine ebenfalls ebene Konvektionsbarriere aufgebracht. Die Konvektionsbarriere kann mit dem Innenvolumen eines Kulturgefäßes in Verbindung stehen.

### Anschließen an externe Komponenten:

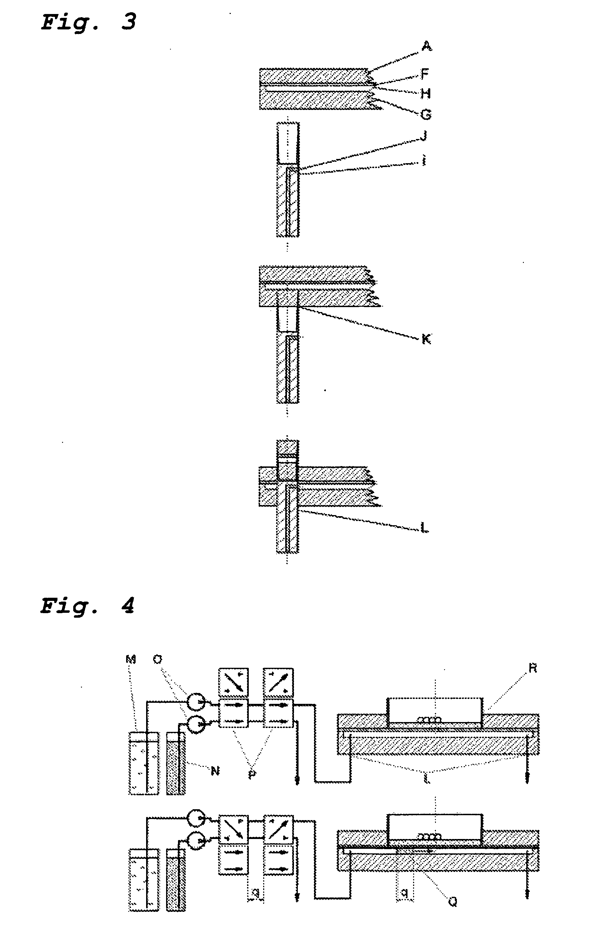

Figur 3 zeigt die Funktion des Stanzwerkzeuges bei der Anbringung von Anschlüssen an die Perfusionsvorrichtung.

Die Kanäle im Substrat des MPA werden durch Ausstanzen eines Teiles des Substrates zur Schnittkante hin geöffnet. Das Stanzwerkzeug enthält mindestens einen Kanal der mit dem anzuschließenden Mikrokanal deckungsgleich positioniert werden kann.

Durch den engen Kontakt zwischen Stanzwerkzeug und Kanal erfolgt die Abdichtung zu allen Seiten hin. Dieses Verfahren soll im Weiteren als Mikrointerface bezeichnet werden.

### Funktion:

Figur 4 zeigt den prinzipiellen Aufbau und die funktionelle Verwendung des MPA.

Ein Transportfluid, welches durch externe Versorgungskomponenten zur Verfügung gestellt wird, strömt über ein Mikrointerface in mindestens einen MPA Kanal über. Über den Kanal dringt das Transportfluid in das MPA vor, bis es schließlich auf eine Öffnung in der Diffusionsbarriere trifft. Über diese Öffnung steht das Transportfluid mit der Konvektionsbarriere in Verbindung. Über den Kanal gelangt das Transportfluid ferner zu einem zweiten Mikrointerface, über welches das Transportfluid wieder das MPA verlässt.

An die Konvektionsbarriere des MPA grenzt das Innenvolumen eines Kulturgefäßes deckungsgleich zu vorhanden Öffnungen in der Diffusionsbarriere. Das Innenvolumen kann durch eine Flüssigkeit ausgefüllt sein, welche sich aus denselben Komponenten wie das Transportfluid zusammensetzt.

Ziel des Transportfluides ist es, ein bestimmtes Volumen einer Perfusionssubstanz einzuschließen und es durch mindestens einen der MPA Kanäle zu transportieren. Dadurch kommt die Perfusionssubstanz über die Öffnungen in der Diffusionsbarriere mindestens einmal mit der Konvektionsbarriere in Kontakt.

Sofern über die Konvektionsbarriere ein Konzentrationsgradient für die Perfusionssubstanz besteht, diffundiert diese aus dem MPA Kanal durch die Konvektionsbarriere in das Innenvolumen des Kulturgefäßes.

Sofern während des Kontaktes zwischen der Konvektionsbarriere und der Perfusionssubstanz ein Druckgradient besteht, wird Perfusionssubstanz konvektiv aus dem MPA-Kanal durch die Konvektionsbarriere in das Innenvolumen des Kulturgefäßes hinein transportiert.

Sofern während des Kontaktes zwischen der Konvektionsbarriere und dem Transportfluid kein Druckgradient besteht, wird Aufgrund eines Druckverlustes über die Konvektionsbarriere, für durch Poren strömende Flüssigkeiten, hervorgerufen durch in den Poren stattfindende Reibung oder elektrostatische Effekte, keine Konvektion stattfinden. Durch die Fließgeschwindigkeit des Transportfluides und das Volumen der eingeschlossen Perfusionssubstanz definiert sich die Kontaktzeit zwischen Konvektionsbarriere und Perfusionssubstanz. Dadurch ist eine zeitliche Abhängigkeit des Konzentrationsgradienten gegeben, was eine zeitlich kontrollierte Perfusion des Kulturgefäßes ermöglicht.

Sofern das MPA über mehr als einen Kanal, sowie über mehr als eine Öffnung in der Diffusionsbarriere verfügt, kann eine multilokale Perfusion mit unterschiedlichen Substanzen simultan oder seriell durchgeführt werden.

### Kanäle:

Das MPA Substrat ist mit Kanälen strukturiert, durch welche ein Transportfluid fließt, um Perfusionssubstanzen mit der Konvektionsbarriere in Kontakt zu bringen. Um Druckunterschiede auf beiden Seiten der Konvektionsbarriere auszuschließen, können die Kanäle wie ein "VenturiRohr" geformt sein. Hierfür muss sich der Kanal an einer Stelle verengen. Diese Verengung befindet sich dort, wo sich auch die Öffnung in der Diffusionsbarriere befindet. Durch die Strömungsgeschwindigkeit des Transportfluides kann somit der Druck an der Öffnung in der Diffusionsbarriere beeinflusst werden.

### Externe Komponenten:

Zum Bereitstellen eines Transportfluides bietet sich ein Vorratsgefäß, mindestens eine Pumpe und ein Umschaltventil vom Typ "Rheodyne™" an.

Das Umschaltventil stellt ein definiertes Volumen zur Verfügung, in das eine Substanz gefüllt werden kann. Über das Umschaltventil kann dieses Volumen durch Einschleifen in den Transportfluidstrom mit dem Transportfluid eingeschlossen werden. Das sich nun im Transportfluidstrom befindliche Substanzvolumen wird mit der Fließgeschwindigkeit des Transportfluides befördert.

In Fig. 4 ist der Mechanismus des benannten Umschaltventils in zwei möglichen Arbeitsstellungen skizziert.

Da Fließgeschwindigkeit und sämtliche Leitungen und Kanäle so ausgelegt sind, dass es zu keinen Turbulenzen kommt, kann es nur zu einer diffusionsbedingten Durchmischung an der Grenzfläche zwischen eingeschlossener Substanz und Transportfluid kommen.

### Materialien:

Das MPA Substrat ist aus einem flexiblen Material gefertigt, welches durch Mikrointerfaces penetriert werden kann. Ferner zeichnet sich das Substrat dadurch aus, dass es sich inert gegenüber dem Transportfluid oder den Perfusionssubstanzen verhält, wie PDMS, PVC, PAA, etc.

Die Diffusionsbarriere ist aus einem Material gefertigt, welches dieselben Eigenschaften wie das MPA Substrat erfüllt, jedoch nicht flexibel sein muss. Zusätzlich zeichnet sich das Material der Diffusionsbarriere dadurch aus, dass es einen sehr kleinen Diffusionskoeffizienten für alle infrage kommenden Perfusionssubstanzen, Transportfluide sowie Substanzen aus dem Kulturgefäß aufweist. Ferner muss es sich außerdem inert gegenüber Substanzen aus dem Kulturgefäß verhalten. Geeignete Materialien für die Diffusionsbarriere sind beispielsweise ausgewählt aus Polyimid, PDMS (Polydimethylsiloxan), Siliziumnitrit (SiNi), Edelmetall (Gold, Platin).

Die Konvektionsbarriere ist aus einem Material gefertigt, welches innert ist, gegenüber dem Transportfluid, infrage kommenden Perfusionssubstanzen und Substanzen, die sich im Kulturgefäß befinden können. Jedoch kann das Material der Konvektionsbarriere mikro- oder nano- porös sein, um einen Übertritt von Perfusionssubstanzen aus MPA Kanälen in das Kulturgefäß zu gewährleisten.

Das Transportfluid kann jedes beliebige Material im flüssigen oder gasförmigen Aggregatzustand sein.

Perfusionssubstanzen liegen als Ionen oder gelöst im flüssigen Aggregatzustand eventuell unter Zuhilfenahme eines Lösungsmittels vor. Die Perfusionssubstanz kann ferner auch im gasförmigen Zustand vorliegen.

Das Mikrointerface muss aus einem Material gefertigt sein, das einerseits inert gegenüber Transportfluid und Perfusionssubstanzen ist und andererseits in seiner Funktion als Stanzwerkzeug hart genug ist, das MPA Substrat sowie die Diffusions- und Konfektionsbarriere zu durchtrennen. Geeignet sind weiterhin beispielsweise Silberstahl, HSS Werkzeugstahl, Keramik, Glas, wobei die genannten Werkstoffe gegebenenfalls mit carbon-likediamonds beschichtet sein können.

Das erfindungsgemäße MPA ermöglicht die raumzeitliche Kontrolle eines Substanzgradienten innerhalb eines Volumens.

Das erfindungsgemäße MPA kommt ohne elektrische Felder oder Heizelemente aus. Somit sind mögliche Einflüsse auszuschließen, welche durch Temperatur oder elektrische Felder hervorgerufen werden könnten. Dies ist von Vorteil sofern Einflüsse von Perfusionssubstanzen untersucht werden sollen, oder sich Einflüsse wie Hitze oder elektrische Felder unerwünscht auf Perfusionssubstanzen auswirken.

Das erfindungsgemäße MPA erlaubt es auch, einen nicht durch Diffusion angetriebenen Stofftransportmechanismus zu erreichen, nämlich durch Konvektion. Die wesentlich schneller ablaufenden Konvektionsvorgänge ermöglichen sehr steile Flanken ansteigender und abfallender Konzentration der Perfusionssubstanz am Untersuchungsobjekt, während simultan ablaufende, langsamere Diffusionsvorgänge die zunächst steilen Flanken abschwächen würden. Ein besonderer Vorteil der vorliegenden Erfindung ist daher die Nutzung von Konvektionsvorgängen zur Perfusion.

Das erfindungsgemäße MPA wird durch mindestens ein Mikrointerface hydraulisch kontaktiert ohne, dass zu diesem Zweck zusätzliche Hohlräume oder Kavitäten im Substrat (MPA) vorgesehen werden müssen. Hierdurch kann effektiv die Ausbildung von Luftbläschen verhindert werden.

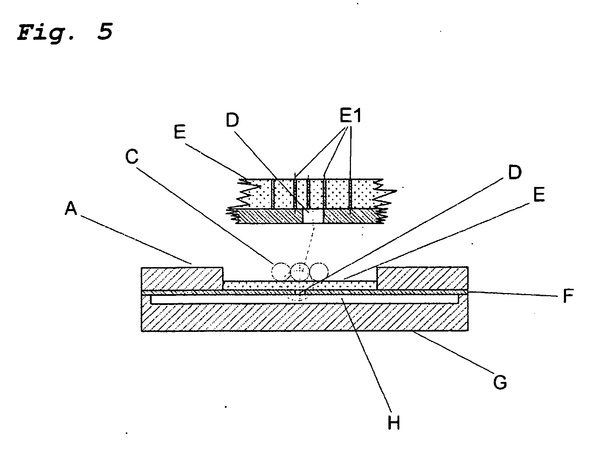

In den Figuren 5 bis 7 sind weitere Ausführungsformen der erfindungsgemäßen Perfusionsvorrichtung näher beschrieben. In diesen Ausführungsformen weist die Konvektionsbarriere besondere Eigenschaften auf, mit denen es möglich ist, einen durch Konvektion angetriebenen Stofftransport zu ermöglichen. In diesen Ausführungsformen werden die folgenden Anforderungen an die Konvektionsbarriere, das Transportfluid, die Perfusionssubstanz und die Flüssigkeit im Kulturgefäß gestellt, welche nachfolgend erläutert werden.

Zwischen dem Stofftransportmechanismus mittels Diffusion und dem mittels Konvektion bestehen erhebliche Unterschiede. Dies wird darin deutlich, dass die wesentlich schneller ablaufenden Konvektionsvorgänge sehr steile Flanken ansteigender und abfallender Konzentration der Perfusionssubstanz am Untersuchungsobjekt ermöglichen, während simultan ablaufende, langsamere Diffusionsvorgänge die zunächst steilen Flanken abschwächen würden.

Weiterhin muss das Material, aus dem die Konvektionsbarriere besteht, so beschaffen sein, dass Perfusionssubstanzen, Transportfluide und sonstige Flüssigkeiten, die mit dem MPA in Kontakt kommen, unlöslich in der Konvektionsbarriere sind.

In der Figur 5 ist gezeigt, dass beide einander gegenüberliegende Seiten der Konvektionsbarriere durch Poren E1 mit definiertem Querschnitt verbunden sind. Dieser Querschnitt ist kleiner als eventuell zur Anwendung kommende ferromagnetische und/oder kolloidale Partikel, jedoch groß genug, um die Konvektion von Perfusionssubstanzen zu gestatten.

Die Innenwände der Poren zeichnen sich dadurch aus, dass Wechselwirkungen wie Reibung und Oberflächeneffekte sowie elektrostatische Effekte mit hindurch tretenden Substanzen auftreten, um einen Druckverlust proportional zur Fließgeschwindigkeit (im Wesentlichen nach dem Gesetz von Hagen-Poiseuille) über die Konvektionsbarriere für diese Substanzen zu erzeugen. Dieser Druckverlust verhindert ungewollte Konvektion.

Konvektion als Mechanismus für den Transport von Perfusionssubstanzen, durch die Konvektionsbarriere hindurch, setzt eine statische Druckdifferenz über die Konvektionsbarriere voraus, damit die Substanzen durch die Poren der Konvektionsbarriere hindurchgelangen. Diese statische Druckdifferenz muss ausreichend dimensioniert sein, um eine hinreichend schnelle Konvektion gegen den Druckverlust in den Poren zu gewährleisten. Eine ausreichend dimensionierte statische Druckdifferenz entspricht mindestens dem Druckverlust in den Poren, da andernfalls jegliche Konvektion effektiv verhindert wird, falls die statische Druckdifferenz kleiner als dieser Druckverlust ist.

Neben einer statischen Druckdifferenz, kann aber auch eine transiente Druckdifferenz ausreichender Höhe und Dauer einen Substanztransport durch die Konvektionsbarriere ermöglichen. Dies ist von entscheidender Bedeutung, da nur so eine raumzeitlich kontrollierte Perfusion der Untersuchungsobjekte mit Perfusionssubstanzen möglich ist.

Mögliche Mechanismen zur Erzeugung einer transienten Druckdifferenz sind:
- osmotischer Druckimpuls
- magnetischer Druckimpuls
- mechanischer Druckimpuls

### Osmotischer Druckimpuls

Nach Hobbie (Hobbie RK. "On the Interpretation of Experiments on Osmotic Pressure", Proc. Nat. Acad. Sci. USA, Vol. 71, No. 8, pp. 3182-3184, August 1974) ist bekannt, dass in einer Flüssigkeit dispergierte kolloidale Partikel genügen, um einen osmotischen Druck über eine semipermeable Membran zu definieren. Die Anforderung an eine solche semipermeable Membran ist erfüllt, sofern beide gegenüberliegende Seiten der Membran durch Poren verbunden sind, deren Querschnitt ein Hindurchtreten von kolloidalen Partikeln mechanisch verhindert, jedoch die Passage von Flüssigkeiten ungehindert gestattet. Ist die Konzentration der kolloidalen Partikel auf einer Seite der Membran höher als auf der gegenüberliegenden Seite, so liegt eine osmotische und somit statische Druckdifferenz vor.

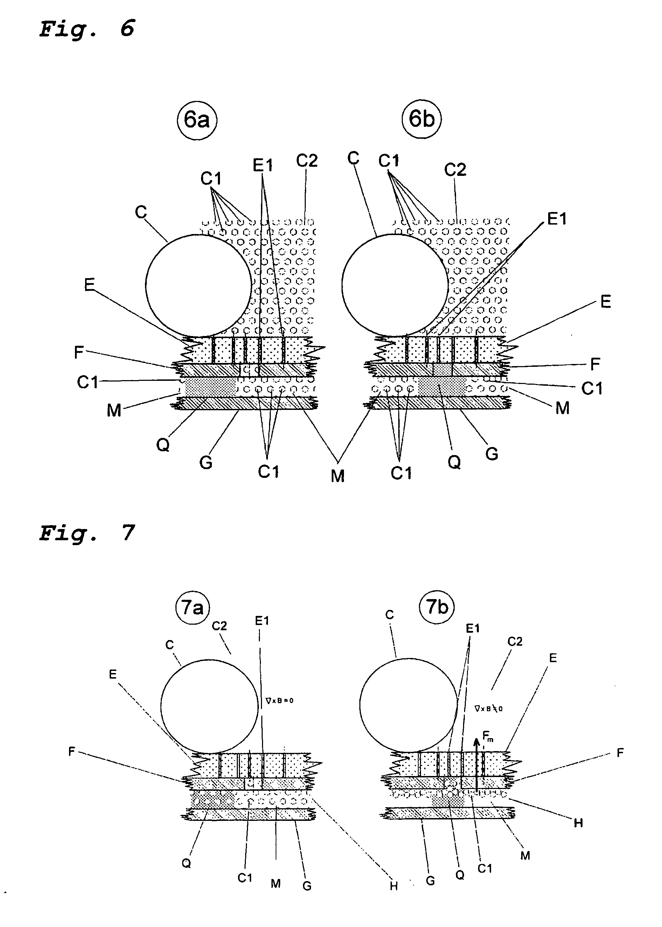

In Figur 6a ist dargestellt, wie dieser Effekt erfindungsgemäß genutzt wird, um Perfusionssubstanz durch die Konvektionsbarriere hindurch zu transportieren. Auf einer Seite der Konvektionsbarriere E befinden sich die Untersuchungsobjekte C sowie kolloidale Partikel C1, die nicht durch die Poren E1 der Konvektionsbarriere hindurchpassen. Solche kolloidalen Partikel C1 sind in der gleichen Konzentration ebenfalls im Transportfluid M dispergiert. Das Transportfluid fließt ständig an der Konvektionsbarriere vorbei und transportiert dabei gleichzeitig ein eingeschlossenes Volumen Perfusionssubstanz Q. Fließgeschwindigkeit und Größe des eingeschlossenen Volumens definieren die Kontaktzeit zwischen Perfusionssubstanz und Konvektionsbarriere. Der Druckverlust, der auf Substanzen (Transportfluid oder Perfusionssubstanzen) in den Poren der Konvektionsbarriere wirkt, schränkt ungewollte Konvektion ein. Die Perfusionssubstanz enthält keine kolloidalen Partikel, weshalb während der Kontaktzeit, im Bereich der fluidischen Kommunikation, zwischen Perfusionssubstanz und Konvektionsbarriere ein osmotisch bedingter, transienter Druckanstieg resultiert wie dies in Figur 6b gezeigt ist.

In Folge dieses Druckanstieges wird der Druckverlust in den Poren überwunden und es kommt zu einem konvektiven Transport der Perfusionssubstanz durch die Konvektionsbarriere hindurch.

Die Kontaktzeit zwischen Perfusionssubstanz und Konvektionsbarriere ist durch die Fließgeschwindigkeit des Transportfluides bestimmt. Ist diese Kontaktzeit verstrichen, so herrscht auf beiden Seiten der Konvektionsbarriere wieder die gleiche Konzentration kolloidaler Partikel. Somit ist die Differenz der osmotischen Drücke verschwunden und der konvektive Transport kommt auf Grund des Druckverlustes in den Poren zum Erliegen.

Anstieg und Abfall der Druckdifferenz während der Kontaktzeit zwischen Perfusionssubstanz und Konvektionsbarriere definieren einen Druckimpuls, der schließlich dem Druckverlust in den Poren entgegenwirkt und folglich den konvektiven Transport von Perfusionssubstanz durch die Poren der Konvektionsbarriere bewirkt.

### Magnetischer Druckimpuls

Aus Hobbie ist auch bekannt, dass auf ferromagnetische und kolloidale Partikel in einem Magnetfeld eine Kraft wirkt. Sind die kolloidalen Partikel gleichzeitig in einer Flüssigkeit dispergiert, so entsteht dort ein magnetischer (Über-)Druck, wo sich die Front kolloidaler Partikel aufgrund der magnetischen Kraftwirkung hinbewegt. Dieser Effekt wird erfindungsgemäß ebenfalls genutzt, um durch Auf- und Abbau eines Magnetfeldes, eine transiente Druckdifferenz über die Konvektionsbarriere zu begründeten. In der Figur 7a ist gezeigt, dass ferromagnetische kolloidale Partikel sowohl im Transportfluid als auch in der Perfusionssubstanz enthalten sind. Da die Poren in der Konvektionsbarriere ein Hindurchtreten von kolloidalen Partikeln verhindern, verbleiben die Partikel auf einer Seite der Konvektionsbarriere. In den Poren wirkt ein Druckverlust auf Substanzen wie Transportfluid, Perfusionssubstanzen oder Nährlösungen, sowie sonstige Flüssigkeiten, wodurch ungewollte Konvektion verhindert wird.

Wie in der Figur 7b dargestellt, wirkt in einem Magnetfeld eine gerichtete Kraft Fₘ (Richtung ist dargestellt) auf die ferromagnetischen und kolloidalen Partikel. Aufgrund dieser Kraftwirkung bewegen sich die ferromagnetischen und kolloidalen Partikel in Richtung der Konvektionsbarriere, wodurch ein Überdruck zwischen der Front sich bewegender Partikel und der Konvektionsbarriere entsteht. Dieser Überdruck ist höher, als der Druckverlust in den Poren, weshalb ein gerichteter konvektiver Substanztransport durch die Konvektionsbarriere hindurch erfolgt.

Vorteilhafterweise wird das Magnetfeld nur solange aufrecht erhalten, wie Perfusionssubstanz mit der Konvektionsbarriere in Kontakt steht und konvektiv transportiert werden soll.

Auch eine ständige Pumpwirkung im dynamischen Gleichgewicht mit kontinuierlich an der Konvektionsbarriere vorbeifließendem Transportfluid/Perfusionssubstanz ist erfindungsgemäß vorgesehen.

Eine vorteilhafte Ausbildung dieser Partikel- getriebenen Pumpe ist weiterhin ein in sich geschlossener Kreislauf, der ein Recycling ferromagnetischer und kolloidaler Partikel ermöglicht.

### Mechanischer Druckimpuls

Zur Überwindung des Druckverlustes, welcher in den Poren auf Substanzen wirkt, kann auch ein mechanischer Druckimpuls genügen, um die Perfusionssubstanz durch die Konvektionsbarriere zu befördern. Dieser Druckimpuls wird erfindungsgemäß durch das Zusammendrücken des Mikrokanals H erzeugt, oder über das externe Versorgungssystem gemäß der Figur 4 und den Mikrokanal H, über den die Perfusionssubstanz an die Konvektionsbarriere gelangt, angelegt.

Erfindungsgemäß weisen die einzelnen Komponenten folgende Eigenschaften auf.

Die Konvektionsbarriere besteht aus einem Material in welchem alle mit dem MPA in Kontakt kommenden Stoffe, insbesondere Transportfluide, Perfusionssubstanzen, Nährlösungen oder anderweitige Flüssigkeiten sowie kolloidale Partikel unlöslich sind. Ferner weist das Material der Konvektionsbarriere Poren auf, die zwei gegenüberliegende Seiten der Konvektionsbarriere miteinander verbinden. Die Poren können als zylinderförmige Löcher oder durch die Struktur des Materials, aus dem die Konvektionsbarriere besteht, definiert sein. Derartige Materialen sind durch Sintern von Glaspartikeln, durch Erschütterungen, Auslaugungsprozesse, Ätzprozesse, auf Partikelbeschuss basierenden Prozesse, Laserätzen, Plasmaätzen und durch biologische Ab- und Aufbauprozesse, beispielsweise so genannter Cocolithe und Frustules aus beispielsweise diatomischer Mikrospezies, erhältlich.

Die kolloidalen Partikel sind biologisch und chemisch inert, weisen eine Größe auf, die größer als die Poren in der Konvektionsbarriere sind. Sie besitzen vorteilhafterweise eine hydrophile Oberfläche und sind gegebenenfalls ferromagnetisch. Vorzugsweise stoßen sich die Partikel voneinander ab.

Das Transportfluid ist biologisch und chemisch inert, vorzugsweise hydrophil und enthält gegebenenfalls eine Dispersion von kolloidalen und/oder ferromagnetischen Partikeln.

Die Perfusionssubstanz ist biologisch oder chemisch aktiv und enthält gegebenenfalls eine Dispersion von kolloidalen und/oder ferromagnetischen Partikeln.

Die Flüssigkeit im Kulturgefäß ist biologisch und chemisch inert, enthält gegebenenfalls die erforderlichen Nährstoffe zum Erhalt der biologischen Materialien, und enthält gegebenenfalls eine Dispersion von kolloidalen Partikeln.

Mit der erfindungsgemäßen Perfusionsvorrichtung ist es möglich die Perfunsionssubstanz in Abhängigkeit von der verwendeten Konvektionsbarriere mittels Konvektion an das biologische Material zu führen. Durch die rasche Anflutung lassen sich bisher nicht zu untersuchende Problemstellungen lösen.

### Bezugzeichenliste

- A: obere Substratschicht mit Öffnung als Kulturgefäß-oder zur Aufnahme eines Kulturgefäßes
- B: Kulturgefäß
- C: biologische Zellen oder anderweitige biologische Objekte
- D: Öffnungen in der Diffusionsbarriere
- E: Konvektionsbarriere
- F: Diffusionsbarriere
- G: untere Substratschicht mit Mikrokanälen
- H: Mikrokanal
- i: Stanzwerkzeug zum Anschließen externer Komponenten an die Mikrokanäle des MPA
- J: Kanal im Stanzwerkzeug
- K: Stanzwerkzeug dringt in das Substrat des MPA ein
- L: Stanzwerkzeug ist so positioniert, dass der Kanal im Stanzwerkzeug mit einem Kanal des MPA in konzentrische Übereinstimmung gebracht ist
- M: Transportfluid
- N: Perfusionssubstanz
- O: Pumpen
- P: Ventilsatz
- Q: In das Transportfluid eingeschlossene Perfusions-substanz
- q: Länge des in das Transportfluid eingeschlossenen Volumens an Perfusionssubstanz
- R: Perfusionsvorrichtung (Mikroperfusionsarray)
- E1: Poren in der Konvektionsbarriere
- C1: Kolloidale Partikel
- C2: Flüssigkeit oder Nährlösung für C
- Fₘ: gerichtete magnetische Kraft

## Patentansprüche

1. Perfusionsvorrichtung (R) zum Perfundieren von biologischem Material (C), aufweisend
- ein Trägersubstrat (G) mit mindestens einem darin befindlichen Kanal (H),
- eine auf dem Trägersubstrat (G) flächig angeordnete Diffusionsbarriere (F), welche die Oberseite der im Trägersubstrat (G) angeordneten Kanäle (H) abdeckt, wobei die Diffusionsbarriere (F) im Bereich der Kanäle (H) mit mindestens einer Perforation (D) versehen ist
- eine auf der Diffusionsbarriere (F) flächig angeordnete Konvektionsbarriere (E),
- je Kanal (H) mindestens je zwei Anschlüsse zum Verteilen von Fluiden in dem Kanal.

2. Perfusionsvorrichtung (R), gemäß Anspruch 1, **dadurch gekennzeichnet, dass** auf der Oberseite der Konvektionsbarriere (E) eine weitere Substratschicht (A) flächig angeordnet ist, wobei in der Substratschicht (A) im Bereich der Perforation der Diffusionsbarriere (F) mindestens eine Öffnung vorgesehen ist.

3. Perfusionsvorrichtung (R), gemäß Anspruch 1 oder 2, **dadurch gekennzeichnet, dass** auf einer Seite der Konvektionsbarriere (E) im wesentlichen konzentrisch zu den Perforationen in der Diffusionsbarriere ein Hohlzylinder als Kulturgefäß (B) angeordnet ist.

4. Perfusionsvorrichtung (R), gemäß einem der voranstehenden Ansprüche, **dadurch gekennzeichnet, dass** das Trägersubstrat (G) und/oder die weitere Substratschicht aus einem flexiblen Material bestehen, welches gegen die Fluide im Kanal (H) chemisch beständig ist.

5. Perfusionsvorrichtung (R), gemäß einem der voranstehenden Ansprüche, **dadurch gekennzeichnet, dass** die Diffusionsbarriere (F) aus einem flexiblen oder starren Material besteht, welches gegen die Fluide im Kanal chemisch beständig ist.

6. Perfusionsvorrichtung (R), gemäß einem der voranstehenden Ansprüche, **dadurch gekennzeichnet, dass** die Konvektionsbarriere (E) aus einem Material besteht, welches gegen die Fluide im Kanal (H) oder im Kulturgefäß (B) chemisch beständig ist.

7. Perfusionsvorrichtung (R), gemäß Anspruch 6, **dadurch gekennzeichnet, dass** die Konvektionsbarriere (E) aus einem mikro- oder nanoporösem Material besteht.

8. Perfusionsvorrichtung (R), gemäß mindestens einem der voranstehenden Ansprüche, **dadurch gekennzeichnet, dass** in den Fluiden im Kanal und/oder im Kulturgefäß dispergierte Partikel vorgesehen sind, welche an der Konvektionsbarriere (E) eine statische oder transiente Druckdifferenz ausbilden und wobei in der Konvektionsbarriere (E) Poren (E1) ausgebildet sind, deren Querschnitt derart gewählt ist, dass der Durchtritt der dispergierten Partikel verhindert, der Durchtritt von Perfusionssubstanzen (Q) jedoch ermöglicht ist.

9. Perfusionsvorrichtung (R), gemäß Anspruch 8, **dadurch gekennzeichnet, dass** die in den Fluiden vorgesehenen dispergierten Partikel aus ferromagnetischen und/oder kolloidalen Partikeln (C1) ausgewählt sind.

10. Perfusionsvorrichtung (R), gemäß Anspruch 8 oder 9, **dadurch gekennzeichnet, dass** die Perfusionsvorrichtung (R) in einem schaltbaren magnetischen Feld angeordnet ist.

11. Perfusionsvorrichtung (R), gemäß einem der voranstehenden Ansprüche, ferner aufweisend ein Werkzeug (i) zum Setzen von Anschlüssen zum Verteilen von Fluiden in einem Kanal der Perfusionsvorrichtung (R), bestehend aus einem im Wesentlichen zylindrischen Körper, der teilweise als Hohlzylinder und teilweise als Vollzylinder ausgebildet ist, wobei der Hohlzylinder als Stanze wirkt und der Vollzylinder eine Bohrung aufweist, welche durch den Boden des zylindrischen Körpers in den Vollzylinder führt und aus der Seitenwand des Vollzylinders austritt.

12. Perfusionsvorrichtung (R) gemäß Anspruch 11 , **dadurch gekennzeichnet, dass** das Werkzeug (i) aus einem Material besteht, welches zum Stanzen der Perfusionsvorrichtung (R) geeignet ist und gegen die Fluide im Kanal chemisch beständig ist.

13. Verfahren zum Perfundieren von biologischem Material (C), wobei man
in einer Perfusionsvorrichtung (R), welche eine mit Poren (E1) versehene Konvektionsbarriere (E)aufweist, welche auf der einen Oberfläche das biologische Material (C) trägt und auf der gegenüberliegenden Oberfläche mit einem Transportfluid (M) in fluidischer Kommunikation ist,
das Transportfluid (M) mit dispergierten Partikeln versieht, welche an der Konvektionsbarriere (E) eine Druckdifferenz ausbilden, da die Poren der Konvektionsbarriere (E) einen Durchmesser aufweisen, der kleiner ist als der Durchmesser der dispergierten Partikel,
dem Transportfluid (M) eine raumzeitlich definierte Menge einer Perfusionssubstanz (N) zusetzt und diese in Richtung auf den Bereich der fluidischen Kommunikation leitet,
und wobei die Perfusionssubstanz (N) beim Erreichen des Bereichs der fluidischen Kommunikation mittels der herrschenden Druckdifferenz durch die Konvektionsbarriere (E) hindurch zum biologischen Material strömt.

14. Verfahren, gemäß Anspruch 13, **dadurch gekennzeichnet, dass** man im Transportfluid (M) als dispergierte Partikel kolloidale Partikel verwendet und
das biologische Material in eine Nährlösung einbringt, welche gleichfalls kolloidale Partikel in einer Menge enthält, die zu einer Druckdifferenz führt, welche den Wert null besitzt.

15. Verfahren, gemäß Anspruch 13, **dadurch gekennzeichnet, dass** die im Transportfluid (M) und/oder in der Perfusionssubstanz (N) dispergierten Partikel ferromagnetische Partikel sind, und wobei man wenigstens im Bereich der fluidischen Kommunikation ein schaltbares und in der Stärke und Richtung regulierbares Magnetfeld anlegt, um die ferromagnetischen Partikel zu bewegen.

## Claims

1. Perfusion device (R) for perfusing biological material (C), comprising
- a carrier substrate (G) with at least one channel (H) located therein,
- a planar diffusion barrier (F) arranged on the carrier substrate (G), which covers the topside of the channels (H) located in the carrier substrate (G), whereby at least one perforation (D) is provided in the diffusion barrier (F) in the region of the channels (H),
- a planar convection barrier (E) arranged on the diffusion barrier (F),
- at least two connections at each channel (H) for distributing fluids in the channel.

2. Perfusion device (R), according to claim 1, **characterized in that** on the topside of the convection barrier (E) a further substrate layer (A) is arranged planar, whereby in the substrate layer (A) in the region of the perforation of the diffusion barrier (F) at least one opening is provided.

3. Perfusion device (R), according to claims 1 or 2, **characterized in that** on one side of the convection barrier (E), substantially concentric in respect of the perforations in the diffusion barrier, a hollow cylinder being a culture vessel (B) is arranged.

4. Perfusion device (R), according to any of the preceding claims, **characterized in that** the carrier substrate (G) and/or the further substrate layer is made of a flexible material, which is chemically stable against the fluids in channel (H).

5. Perfusion device (R), according to any of the preceding claims, **characterized in that** the diffusion barrier (F) is made of a flexible or rigid material which is chemically stable against the fluids in the channel.

6. Perfusion device (R), according to any of the preceding claims, **characterized in that** the convection barrier (E) is made of a material which is chemically stable against the fluids in channel (H) or in the culture vessel (B).

7. Perfusion device (R), according to claim 6, **characterized in that** the convection barrier (E) is made of a micro- or nanoporous material.

8. Perfusion device (R), according to any of the preceding claims, **characterized in that** in the fluids in the channel and/or in the culture vessel dispersed particles are provided, which at the convection barrier (E) generate a static or transient pressure difference and whereby in the convection barrier (E) pores (E1) are formed, the cross section thereof is selected such that the passage of the dispersed particles is prevented but the passage of perfusion substances (Q) is enabled.

9. Perfusion device (R), according to claim 8, **characterized in that** the dispersed particles provided in the fluids are selected from ferromagnetic and/or colloidal particles (C1).

10. Perfusion device (R), according to claim 8 or 9, **characterized in that** the perfusion device (R) is arranged in a switchable magnetic field.

11. Perfusion device (R), according to any of the preceding claims, furthermore bearing a tool (i) for placing connections for distributing fluids in a channel of the perfusion device (R), composed of a substantially cylindrical body, which in part is in form of a hollow cylinder and in part in form of a solid cylinder, whereby the hollow cylinder functions as a punch and the hollow cylinder has a bore, which passes through the bottom of the cylindrical body into the solid cylinder and passes out through the side wall of the solid cylinder.

12. Perfusion device (R), according to claim 11, **characterized in that** the tool (i) is made of a material which is suitable for punching the perfusion device and is chemically stable against the fluids in the channel.

13. Method for the perfusion of biological material (C), whereby
in a perfusion device (R), which bears a convection barrier provided with pores (E1), which on one surface carries the biological material (C) and on the opposing surface is in fluid communication with a transport fluid (M),
the transport fluid (M) is provided with dispersed particles, which at the convection barrier (E) produce a pressure difference, because the pores of the convection barrier (E) have a diameter that is smaller than the diameter of the dispersed particles,
adding to the transport fluid (M) a spatiotemporal defined amount of a perfusion substance (N) and leading it in the direction of the region of fluid communication,
and whereby the perfusion substance (N) on reaching the region of fluid communication by means of the pressure difference present flows through the convection barrier (E) to the biological material.

14. Method according to claim 13, **characterized in that** in the transport fluid (M) colloidal particles are used as dispersed particles and the biological material is introduced into a nutrient solution, which equally contains colloidal particles in an amount, that leads to a pressure difference that has the value zero.

15. Method, according to claim 13, **characterized in that** in the particles dispersed in the transport fluid (M) and/or in the perfusion substance (N) are ferromagnetic particles, and whereby at least in the region of fluid communication a switchable and in respect of strength and direction adjustable magnetic field is applied, in order to move the ferromagnetic particles.

## Revendications

1. Dispositif de perfusion (R) pour la perfusion d'un matériel biologique (C), présentant
- un substrat de support (G) avec au moins un canal (H) se trouvant à l'intérieur,
- une barrière de diffusion (F) disposée à plat sur le substrat de support (G), qui recouvre le dessus des canaux (H) disposés dans le substrat de support (G), où la barrière de diffusion (F) est pourvue dans la zone des canaux (H) d'au moins une perforation (D),
- une barrière de convection (E) disposée à plat sur la barrière de diffusion (F),
- pour chaque canal (H) au moins deux raccords pour la distribution de fluides dans le canal.

2. Dispositif de perfusion (R) selon la revendication 1, **caractérisé en ce que** sur le dessus de la barrière de convection (E) est disposée à plat une autre couche de substrat (A), où au moins une ouverture est prévue dans la couche de substrat (A) dans la zone de la perforation de la barrière de diffusion (F).

3. Dispositif de perfusion (R), selon la revendication 1 ou 2, **caractérisé en ce que** sur un côté de la barrière de convection (E) est disposé un cylindre creux en tant que récipient de culture (B) pour l'essentiel concentriquement par rapport aux perforations dans la barrière de diffusion.

4. Dispositif de perfusion (R) selon l'une des revendications précédentes, **caractérisé en ce que** le substrat de support (G) et/ou l'autre couche de substrat se composent d'un matériau souple qui résiste chimiquement aux fluides dans le canal (H).

5. Dispositif de perfusion (R) selon l'une des revendications précédentes, **caractérisé en ce que** la barrière de diffusion (F) se compose d'au matériau souple ou rigide qui résiste chimiquement aux fluides dans le canal.

6. Dispositif de perfusion (R) selon l'une des revendications précédentes, **caractérisé en ce que** la barrière de convection (E) se compose d'un matériau, qui résiste chimiquement aux fluides dans le canal (H) ou dans le récipient de culture (B).

7. Dispositif de perfusion (R) selon la revendication 6, **caractérisé en ce que** la barrière de convection (E) se compose d'un matériau micro- ou nanoporeux.

8. Dispositif de perfusion (R) selon au moins l'une des revendications précédentes, **caractérisé en ce que** dans les fluides dans le canal et/ou dans le récipient de culture sont prévues des particules dispersées qui forment sur la barrière de convection (E) une différence de pression statique ou transitoire et où des pores (E1) sont formés dans la barrière de convection (E), pores (E1) dont la section transversale est choisie de manière à ce que le passage des particules dispersées soit empêché, mais que le passage des substances de perfusion (Q) soit rendu possible.

9. Dispositif de perfusion (R) selon la revendication 8, **caractérisé en ce que** les particules dispersées prévues dans les fluides sont choisies parmi les particules ferromagnétiques et/ou colloïdales (C1).

10. Dispositif de perfusion (R) selon la revendication 8 ou 9, **caractérisé en ce que** le dispositif de perfusion (R) est disposé dans un champ magnétique commutable.

11. Dispositif de perfusion (R) selon l'une des revendications précédentes, présentant en outre un outil (i) pour placer des raccords pour la distribution de fluides dans un canal du dispositif de perfusion (R), se composant d'un corps pour l'essentiel cylindrique, qui est configuré partiellement en tant que cylindre creux et partiellement en tant que cylindre plein, où le cylindre creux fonctionne comme une presse à poinçonner et le cylindre plein présente un alésage qui conduit dans le cylindre plein à travers la base du corps cylindrique et ressort par la paroi latérale du cylindre plein.

12. Dispositif de perfusion (R) selon la revendication 11, **caractérisé en ce que** l'outil (i) se compose d'un matériau qui est approprié pour le poinçonnage du dispositif de perfusion (R) et résiste chimiquement aux fluides dans le canal.

13. Procédé de perfusion d'un matériel biologique (C), où
dans un dispositif de perfusion (R), qui présente une barrière de convection (E) pourvue de pores (E1), qui supporte sur une surface le matériel biologique (C) et qui est sur la surface opposée en communication fluidique avec un fluide de transport (M),
le fluide de transport (M) est pourvu de particules dispersées qui forment une différence de pression sur la barrière de convection (E), étant donné que les pores de la barrière de convection (E) présentent un diamètre qui est inférieur au diamètre des particules dispersées,
une quantité définie dans le temps et l'espace d'une substance de perfusion (N) est ajoutée au fluide de transport (M) et elle est dirigée dans la direction de la zone de la communication fluidique,
et où la substance de perfusion (N) lorsque la zone de la communication fluidique est atteinte au moyen de la différence de pression régnante s'écoule à travers la barrière de convection (E) vers le matériel biologique.

14. Procédé selon la revendication 13, **caractérisé en ce que** des particules colloïdales sont utilisées comme particules dispersées dans le fluide de transport (M) et le matériel biologique est introduit dans un bouillon de culture, qui contient également des particules colloïdales en une quantité qui conduit à une différence de pression, laquelle possède la valeur zéro.

15. Procédé selon la revendication 13, **caractérisé en ce que** les particules dispersées dans le fluide de transport (M) et/ou dans la substance de perfusion (N) sont des particules ferromagnétiques et où au moins dans la zone de la communication fluidique un champ magnétique commutable et modulable en termes de puissance et de direction est établi afin de déplacer les particules ferromagnétiques.
